# EUROPÄISCHE PATENTSCHRIFT

(11) **EP 0 524 441 B1**
(45) Veröffentlichungstag und Bekanntmachung des Hinweises auf die Patenterteilung: **29.11.1995**
(21) Anmeldenummer: 92110526.8
(22) Anmeldetag: 23.06.1992
(51) Int. Cl.: F16B 7/04, F16B 2/06, A61B 17/60

(54) **Klemmverbindung zum Verbinden zweier Konstruktionselemente für eine, insbesondere osteosynthetische Fixationsvorrichtung**
Clamping joint for connecting two construction elements, in particular for an osteosynthetic fixator
Joint de serrage pour joindre deux éléments de construction, en particulier pour un fixateur ostéosynthétique

(30) Priorität: 23.07.1991 CH 2195/91
(43) Veröffentlichungstag der Anmeldung: 27.01.1993
(73) Patentinhaber: Synthes AG, Chur, CH-7002 Chur (CH)
(72) Erfinder: Schläpfer, Johannes Fridolin, Dr., CH-8750 Glarus (CH); Hess, Martin, CH-4434 Hölstein (CH); Woreth, Roland, CH-4144 Arlesheim (CH); Tanner, Peter, CH-4416 Bubendorf (CH); Weigum, Hans, CH-4435 Niederdorf (CH)
(74) Vertreter: Lusuardi, Werther Giovanni, Dr.

(56) Entgegenhaltungen:
- WO-A-88/01152
- DE-B- 1 045 181
- DE-U- 9 101 321
- US-A- 2 831 717
- US-A- 3 677 584

## Beschreibung

Die Erfindung betrifft eine Klemmverbindung zum Verbinden zweier Konstruktionselemente für eine, insbesondere osteosynthetische Fixationsvorrichtung gemäss den Merkmalen des Anspruchs 1.

Bei einer bekannten Klemmverbindung (DE-GM 91 01 321) sind zwei annähernd gleiche Klemmelemente und eine dazwischen befindliche Druckscheibe vorgesehen, welche allesamt von einer zentral zugeordneten Gewindeschraube des Verspannmittels durchdrungen sind. Letzteres liegt auf dem oberen Klemmelement auf und ist in das untere Klemmelement eingeschraubt. Die Klemmelemente haben dabei zwei jeweils halbzylindrische Ausnehmungen, die zur Aufnahme von als Stäbe ausgebildeten Konstruktionselementen dienen. Mit dieser Klemmverbindung können zwei solche Konstruktionselemente fest miteinander verbunden werden, indem jeweils zwischen ein Klemmelement und die Druckscheibe ein Stab, beispielsweise ein Kohlenfaserstab und eine Schanzsche Schraube, eingeführt und durch die einen Drehknopf aufweisende Gewindeschraube festgeklemmt werden. Die auf den Stab wirkende effektive Klemmkraft hängt von dem Verhältnis des Abstandes zwischen der Achse des Verspannmittels und der des Stabes zu dem Abstand zwischen der Verspannmittelachse und der Auflage des Klemmelementes der Druckscheibe ab. Um bei gegebener Verspannkraft eine möglichst grosse effektive Klemmkraft auf den Stab zu erzielen, muss nach dem Hebelgesetz das obengenannte Verhältnis möglichst klein gewählt werden. Dies hat zur Folge, dass die Klemmelemente zwangsweise in ihrer Dimension grösser werden. Diese insbesondere für eine externe Fixation im osteosynthetischen Anwendungsbereich benützten Klemmverbindungen werden vornehmlich für die Fixation von kleineren Körperteilen verwendet, bei der die Klemmelemente nicht zu gross dimensioniert sein dürfen. In der Praxis hat sich gezeigt, dass aus den genannten Gründen mit solchen beschriebenen Klemmverbindungen eine zu wenig solide Klemmkraft und damit keine sichere Verspannung zwischen den Konstruktionselementen beziehungsweise der gesamten Fixationsvorrichtung erzielt wird. Ausserdem kommt es öfters vor, dass die Spannkraft nach einer gewissen Zeit nachlässt und somit eine Fixation schlechthin nicht mehr gegeben ist.

Bei einer Klemmvorrichtung gemäss der WO-A1 88/01152 sind ähnlich wie bei der oben beschriebenen, vorbekannten Klemmverbindung Klemmscheiben vorgesehen, die paarweise jeweils eine Schraube oder dergleichen in halbrunden Ausnehmungen positionieren und mittels einer ihnen axial zugeordneten Schraube festklemmen. Zum Verbinden zweier Konstruktionselemente sind für eine Verspannschraube zwei Paare solcher Klemmscheiben vorgesehen. Diese Klemmvorrichtung hat im Prinzip dieselben Nachteile wie die oben beschriebene, denn die effektiv auf einen Stab wirkende Klemmkraft geht genauso von einem ungünstigen Verhältnis des Abstandes zwischen der Achse der Verspannschraube und der Auflage der Klemmscheiben aus. Daran ändert sich auch nichts durch die Konstruktion der Klemmscheiben an der Auflage mit dem vorgesehenen gegenseitigen zahnförmigen Eingriff. Bei diesem bekannten, offenen System wird die vorhandene schiefe Ebene nicht zur Verbesserung der Klemmung, sondern einzig zur Verbesserung der Zentrierung zwischen Verbindungs- und Klemmelement benutzt.

Schliesslich ist aus der US-A 3,677,584 SHORT eine Klemmvorrichtung bekannt, mit welcher zwei Längsträger in verschiedenen Richtungen zueinander lösbar fixiert werden können, wobei die beiden Stäbe durch Bohrungen eingefädelt werden müssen.

Um diese Nachteile zu beheben, liegt der vorliegenden Erfindung die Aufgabe zugrunde, eine Klemmverbindung der eingangs beschriebenen Gattung zu schaffen, mit der bei einfacher Handhabung eine genügend starke und über längere Zeitdauer annähernd konstant wirksame Klemmkraft der zu verbindenden Konstruktionselemente erzeugt wird.

Die Erfindung löst die gestellte Aufgabe mit einer Klemmverbindung, welche die Merkmale des Anspruchs 1 aufweist.

Eine solche erfindungsgemässe Klemmverbindung hat insbesondere den Vorteil, dass mit ihr bei gleicher Dimensionierung und bei gleicher Verspannkraft wie bei der nach dem Stand der Technik eine bedeutend höhere resultierende Klemmkraft erzielt wird. Zudem ist diese Klemmverbindung in ihrer Ausbildung sehr elastisch, wodurch sich die Klemmverbindung bei Erschütterungen oder dergleichen nicht selbsttätig löst und somit die Langzeitwirkung der Verspannung erhalten bleibt.

Zur Erzielung einer maximalen auf das Konstruktionselement wirkenden Klemmkraft soll das Verhältnis des Abstandes zwischen der Achse des Konstruktionselementes und der des Verspannmittels zu dem Abstand zwischen der Achsmitte des Konstruktionselementes und der Wirkungslinie der Auflagekraft weniger als 1,0 betragen und vorzugsweise zwischen 0,6 und 0,1 liegen.

Die Spannflächen des Verbindungs- und Konstruktionselementes, respektive die Auflageflächen des Klemm- und des Verbindungselementes sind zwecks gegenseitig annähernd reibungsfreier Auflage in ihrer Oberflächenbeschaffenheit möglichst glatt vorgesehen.

Das Verbindungselement kann zylindrisch, rechteckig oder andersförmig ausgebildet sein und hat an seinen Stirnflächen je eine gleichachsige Gewindeschraube, wobei auf letzterer jeweils ein kappenförmiges Klemmelement angeordnet ist, das eine Spannkraft auf das dazwischengeklemmte Konstruktionselement ausübt und gegenüberliegend mit seiner Auflagefläche parallel zur Verspannrichtung an der Auflagefläche des Verbindungselementes aufliegt. Je nachdem sind diese korrespondierenden Auflageflächen zylindrisch oder eben ausgebildet.

Die Erfindung sowie weitere Vorteile derselben sind in den nachfolgenden Ausführungsbeispielen anhand der Zeichnung näher erläutert. Es zeigt:
Fig. 1 einen teilweisen Längsschnitt durch eine erfindungsgemässe Klemmverbindung;
Fig. 2 einen Querschnitt II-II nach der Fig. 1;
Fig. 3 einen Längsschnitt einer Klemmverbindung mit einer Pedikelschraube;
Fig. 4 einen Längsschnitt einer weiteren Variante einer teilweise dargestellten erfindungsgemässen Klemmverbindung;
Fig. 5 und Fig. 6 je eine perspektivische Ansicht einer Variante einer Klemmverbindung; und
Fig. 7a und 7b einen Längsschnitt durch eine weitere Variante einer teilweise dargestellten erfindungsgemässen Klemmverbindung.

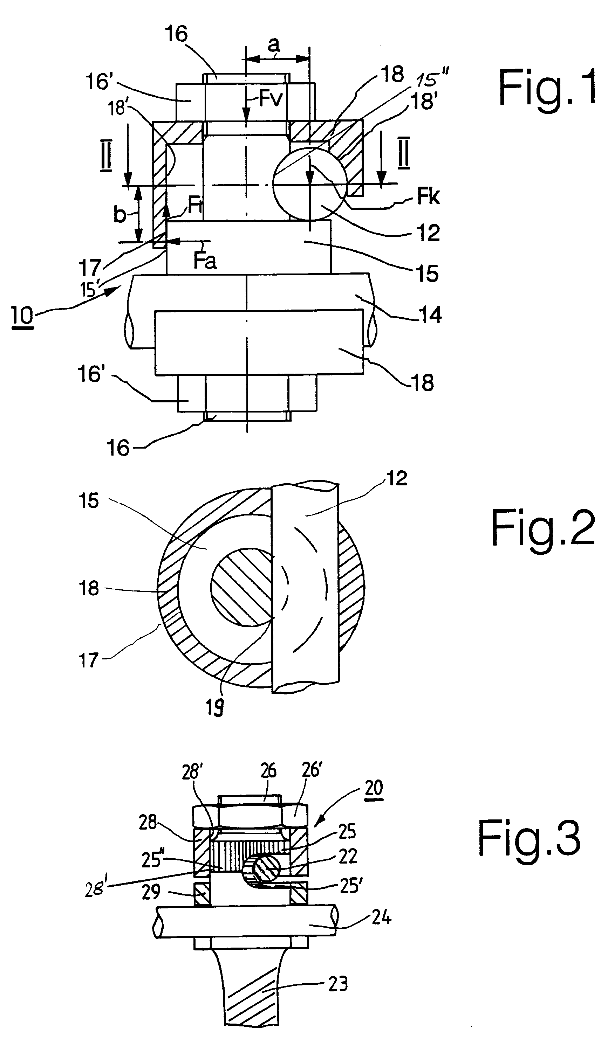

Fig. 1 zeigt eine Klemmverbindung 10 als Bestandteil einer nicht näher dargestellten Fixationsvorrichtung, die zwei Konstruktionselemente 12 und 14 zueinander fixiert. Bei letzteren handelt es sich beispielsweise zum einen um einen Verbindungsstab und zum anderen um eine Schanzsche Schraube, welche zum Beispiel in einen Teil eines gebrochenen Fingerknochens eingeschraubt ist. Eine zweite Klemmverbindung kann dann in entsprechender Weise eine solche Schraube im anderen Knochenteil mit dem genannten Verbindungsstab fest verbinden.

Die Klemmverbindung 10 besteht aus einem Verbindungselement 15, aus beidseitig an diesem vorgesehenen Verspannmitteln 16, 16' sowie aus zwei kappenförmigen Klemmelementen 18. Die Verspannmittel setzen sich je aus einer koaxial zum Verbindungselement 15 vorgesehener Gewindeschraube 16 sowie aus einer das Klemmelement 18 verschiebender Mutter 16' zusammen. Neben dem jeweiligen Verspannmittel 16, 16' ist ein dazu quer angeordnetes Konstruktionselement 12, 14 zwischen dem Verbindungselement 15 und dem Klemmelement 18 eingespannt, wobei das Konstruktionselement 12 sowie die mit ihm jeweils zur Anlage kommenden Spannflächen 15' resp. 18' der genannten Elemente eine möglichst glatte Oberfläche aufweisen, damit nicht unerwünschte Reibkräfte auftreten.

Erfindungsgemäss liegt das Klemmelement 18 parallel zur Verspannrichtung am Verbindungselement 15 auf und es wird dabei durch das durch die Verspannkraft Fv erzeugte Moment an das Verbindungselement 15 mit einer senkrecht zur Verspannkraft Fv wirkenden Auflagekraft Fa angedrückt, welche eine Reibkraft Fr zwischen dem Klemm- und Verbindungselement bewirkt, die als einzige die Verspannkraft Fv auf das jeweilige Konstruktionselement 12 reduziert. Die Reibkraft Fr ist bei der vorliegenden Erfindung einerseits um den Faktor des Reibungskoeffizienten kleiner; andererseits soll das Verhältnis des Abstandes a zwischen der Achse des Konstruktionselementes 12 und der des Verspannmittels 16 zu dem Abstand b zwischen der Achse des Konstruktionselementes 12 und der Wirkungslinie der Auflagekraft Fa kleiner als 1,0 und vorzugsweise zwischen 0,6 und 0,1 betragen.

Dies kann erzielt werden, ohne dass das Klemmelement und damit die gesamte Klemmverbindung 10 im Durchmesser vergrössert werden muss.

Gemäss Fig. 2 umgreift das zylindrisch ausgebildete Klemmelement 18 einerseits das Konstruktionselement 12 und andererseits das Verbindungselement 15. Die Gewindeschraube 16 im Zentrum des Verbindungselementes 15 hat fernerhin eine quere Nut 19, in welche das Konstruktionselement 12 hineinragt. Dies führt zu einer Verringerung der Dimension der Klemmverbindung 10. Das Klemm- und Verbindungselement könnten im Querschnitt auch rechteckig oder andersförmig ausgebildet sein.

Fig. 3 zeigt eine Klemmverbindung 20, die aus einem zylindrischen Verbindungselement 25 mit einer Pedikelschraube 23, aus zwei auf diesem axial verschiebbar geführten, ringförmigen Klemmelementen 28, 29 sowie aus einem Verspannmittel 26, 26' besteht. Letzteres hat wiederum eine zum Verbindungselement 25 gehörige Gewindeschraube 26 sowie eine auf dieser aufgeschraubte Mutter 26', die mit dem oberen Klemmelement 28 zur Anlage kommt. Das Klemmelement 28 drückt beim Verspannen auf ein als Querstab ausgebildetes Konstruktionselement 22, das hinwiederum auf das zweite Klemmelement 29 und dieses auf ein Konstruktionselement 24 drückt, das am Verbindungselement 25 aufliegt und wie auch das andere in einer nutförmigen Ausnehmung 25' desselben befindlich ist. Die beiden Ausnehmungen 25' sind übereinander und zueinander versetzt in einem Winkel von 90° angeordnet und zumindest die eine ist derart ausgebildet, dass der darin befindliche Querstab um einige Winkelgrade, z.B. 40°, zum Verbindungselement 25 eingestellt werden kann. Die koaxial am Verbindungselement 25 befindliche Pedikelschraube 23 ist dabei in einen Knochen oder dergleichen eingeschraubt, während der Querstab (Konstruktionselement 22) und das andere Konstruktionselement 24 (Längsträger) als Bestandteil einer Fixationsvorrichtung mit weiteren Klemmverbindungen verbunden sind. Die Oberflächen 28', 25'' des Verbindungselementes 25 und des Spannelementes 28 sind rotationssymmetrisch und in der Kontaktzone, vorzugsweise in Form einer Verzahnung, derart ausgebildet, dass sie in der Verspannrichtung annähernd reibungsfrei gegeneinander verschiebbar aber nicht verdrehbar sind. Die Lage des Konstruktionselementes 22 zum Verbindungselement 25 ist durch eine verdrehsichere Gestaltung der Innenfläche des Klemmelementes 28 und der Aussenfläche des Verbindungselementes 25, vorzugsweise durch Längsverzahnung oder durch eine nicht kreiszylindrische Geometrie des Verbindungselementes 25 und des Klemmelementes 28, vorzugsweise in polygonaler (z.B. hexagonaler) Form, gegeben.

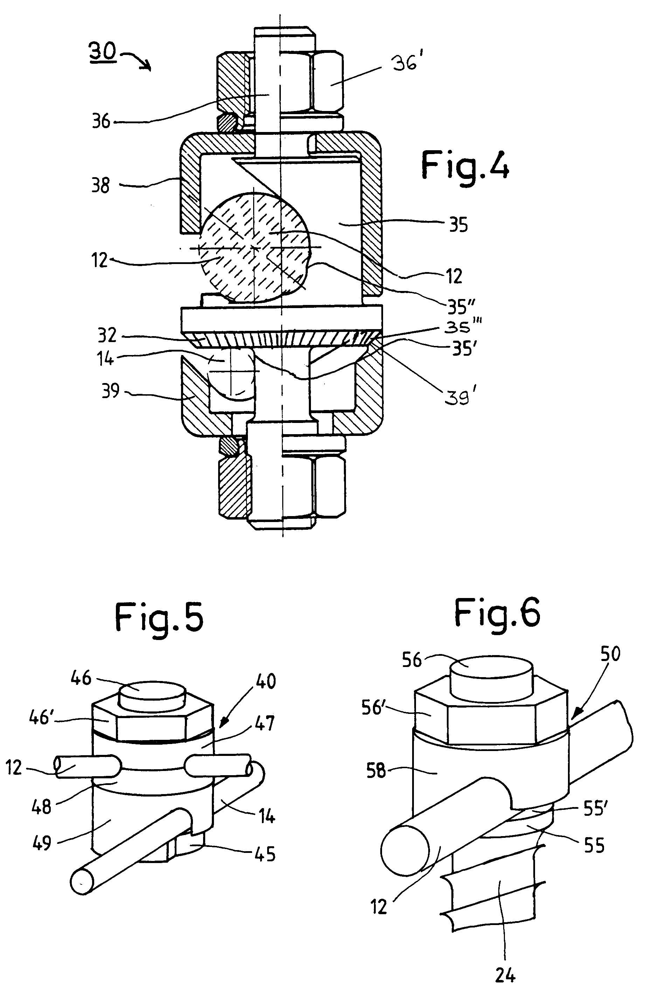

Eine Klemmverbindung 30 nach der Fig. 4 umfasst zwei Klemmelemente 38, 39 und ein im wesentlichen zylindrisches Verbindungselement 35 mit beidseitig koaxial zu diesem vorgesehenen als Verspannmittel dienende Schrauben 36 sowie mit Ausnehmungen 35', 35'' zur Auflage je eines Konstruktionselementes 12, 14. Die eine seitliche Ausnehmung 35'' ist dabei derart gestaltet, dass es durch deren annähernd halbrunde Ausbildung eine Dreipunktauflage in verspanntem Zustand des Konstruktionselementes 12 bewirkt. Vorteilhaft ist letzteres rohrförmig ausgebildet, damit es zwecks Erhöhung der Klemmwirkung eine gewisse Deformation und Anpassung an die Ausnehmung 35'' erfährt. Das andere Konstruktionselement 14 ist von dem eine Längsnut bildenden Klemmelement 39 in eine annähernd rechtwinklige Ausnehmung 35' des Verbindungselementes gedrückt. Das Klemmelement 39 sowie das Verbindungselement 35 sind gegenüberliegend mittels je einer kreisrunden Schrägverzahnung 32 gegeneinander drehgesichert, dagegen in Verspannrichtung möglichst reibungsfrei anliegend angeordnet.

In Fig. 5 ist nochmals eine Klemmverbindung 40 zum Verbinden insbesondere einer Schanzschen Schraube 12 mit einem Querstab 14 für eine externe Fixation gezeigt. Im Unterschied zu derjenigen nach Fig. 1 ist für beide zu klemmenden Stäbe nur ein Verspannmittel 46, 46' vorgesehen. Eine Mutter 46' kommt mit einem oberen Klemmelement 47 zur Anlage, das eine längliche Ausnehmung für die Schraube 12 hat. Die Schraube 12 ihrerseits liegt auf einem auf dem unteren Klemmelement 49 anliegenden Zwischenring 48. Letzterer bewirkt eine Verspannkraft auf das Klemmelement 49, welches den Querstab 14 an das Verbindungselement 45 klemmt. Die beiden Klemmelemente 47 und 49 liegen gegenüber der Stäbe parallel zur Verspannrichtung auf dem Verbindungselement 45 auf, wodurch die erwähnte Reibkraft als reduzierte Gegenkraft beim Verklemmen entsteht.

Die Klemmverbindung 50 gemäss Fig. 6 unterscheidet sich von der nach Fig. 3 nur gerade dadurch, dass das Verbindungselement 55 eine einzige quere Ausnehmung 55' für ein Konstruktionselement 12 aufweist. Ansonsten kann wiederum mittels eines Verspannmittels 56, 56' eine Verspannkraft über das Klemmelement 58 auf das Konstruktionselement 12 ausgeübt und eine Verklemmung desselben bewirkt werden. Im übrigen besitzt das zylindrische Verbindungselement 55 eine koaxiale Pedikelschraube 24.

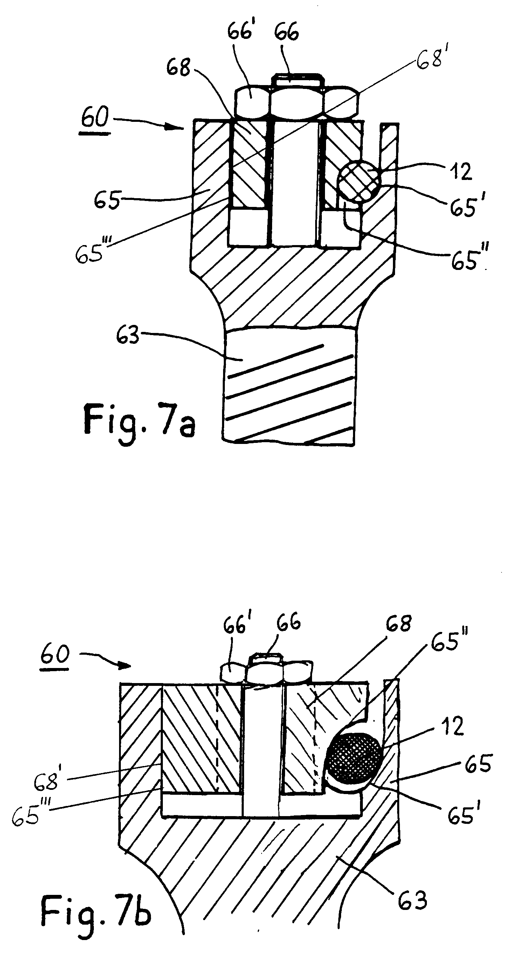

Die Klemmverbindung 60 nach den Fig. 7a und 7b umfasst ein im wesentlichen zylindrisches Verbindungselement 65 mit einer Pedikelschraube 63 sowie mit einer zylindrischen Ausnehmung 65', in der eine koaxiale Gewindeschraube 66 angeordnet ist und darauf ein in der Ausnehmung 65' passendes aussen zylindrisches Klemmelement 68 mittels einer dieses verschiebenden Mutter 66' verstellbar angeordnet ist. Ein seitlich und quer zur Achse der Gewindeschraube 66 vorgesehenes Konstruktionselement 12 , das in einen aussermittigen Schlitz des Verbindungselementes 65 angeordnet ist, ist von der vom Verspannmittel 66, 66' auf das Klemmelement 68 erzeugten Verspannkraft an eine innere Auflagefläche 65' des Verbindungselementes 65 geklemmt. Demnach liegt das Klemmelement 68 entsprechend der Erfindung parallel zur Verspannrichtung am Verbindungselement 65 auf, wodurch die angestrebte maximal mögliche wirksame Klemmkraft auf das Konstruktionselement 12 wiederum erreicht ist.
Bei dieser Ausführungsform können die Ausnehmung 65'' und die innere Auflagefläche 65' auf zwei verschiedene Arten ausgebildet sein. In Fig. 7a entsprechen die Elemente 65' und 65'' der Form des Konstruktionselement 12; in Fig. 7b sind sie derart gestaltet, dass sie zusammen mit dem Klemmelement 68 eine Dreipunktauflage bewirken.

Die Erfindung liesse sich selbstverständlich noch in anderen Varianten darlegen. Es sei nur nebenbei vermerkt, dass als Verspannmittel anstelle einer Schrauben/Mutter-Verbindung grundsätzlich auch ein anderes bekanntes Prinzip verwendbar wäre.

## Patentansprüche

1. Klemmverbindung (10,20) zur Fixierung eines Längsträgers (12,14,22,24), insbesondere einer osteosynthetischen Fixationsvorrichtung, mit
A) einem, eine axiale Durchgangsbohrung aufweisenden Klemmelement (18,28,29,38,48,49,58,68), welches eine Ausnehmung zur Aufnahme des Längsträgers (12,14,22,24) besitzt;
B) einem Verbindungselement (15,25,35,45,55,65) mit einem Grundkörper und einem axial angeordneten, durch die Durchgangsbohrung des Klemmelementes (18,28,29,38,48,49,58,68) führbaren Längskörper, wobei
C) das Verbindungselement (15,25,35,45,55,65) eine Kontaktfläche (15',25'',35'',65''') besitzt, welche in lateralem Kontakt mit einer Kontaktfläche (18',28',39',68') des Klemmelementes (18,28,29,38,48,49,58,68) bringbar ist;
D) Verspannmittel (16,16',26,26'36,36',46,46',56,56',66,66') vorgesehen sind, welche verschieblich auf dem Längskörper des Verbindungselementes (15,25,35,45,55,65) angeordnet und gegen letzteres verspannbar sind;
E) das Klemmelement (18,28,29,38,48,49,58,68) den in seine Ausnehmung eingeführten Längsträger (12,14,22,24) gegen den Grundkörper des Verbindungselementes (15,25,35,45,55,65) drückt und gleichzeitig die in lateralem Kontakt stehenden Kontaktflächen (15',25''',35''',65''';18',39',68',28') gegeneinander presst;
F) das Klemmelement (18,28,29,38,48,49,58,68) dabei durch das durch die Verspannkraft (Fv) erzeugte Moment an das Verbindungselement (15,25,35,45,55) mit einer senkrecht zur Verspannkraft (Fv) wirkenden Auflagekraft (Fa) angedrückt wird, welche eine Reibkraft (Fr) zwischen dem Klemm- und Verbindungselement bewirkt, die als einzige die Verspannkraft (Fv) auf dem Längsträger (12,14,22,24) reduziert; und
G) das Verbindungselement (15,25,35,45,55) eine nutförmige Ausnehmung (19;25') zur Aufnahme des Längsträgers (12,14,22,24) aufweist.

2. Klemmverbindung (10,20) nach Anspruch 1, dadurch gekennzeichnet, dass zur Erzielung einer möglichst grossen auf den Längsträger (12,14,22,24) wirkenden Klemmkraft (Fk) das Verhältnis des Abstandes (a) zwischen der Achse des Längsträgers (12,14,22,24) und der des Verspannmittels (16,16',26,26') zu dem Abstand (b) zwischen der Achse des Längsträgers (12,14,22,24) und der Wirkungslinie der Auflagekraft (Fa) kleiner als 1 beträgt.

3. Klemmverbindung (10,20) nach Anspruch 2, dadurch gekennzeichnet, dass der Abstand (b) grösser als der halbe Durchmesser des Längsträgers (12,14,22,24) ist.

4. Klemmverbindung (10) nach einem der Ansprüche 1 - 3, dadurch gekennzeichnet, dass die Kontaktflächen (18';15') des Verbindungs- (15) und Klemm-Elementes (18) in der Kontaktzone (17) zwecks gegenseitig annähernd reibungsfreiem Gleiten glatt ausgebildet sind.

5. Klemmverbindung (20,30) nach einem der Ansprüche 1 - 3, dadurch gekennzeichnet, dass die Kontaktflächen (28',25',32) des Verbindungs- (25,35) und Klemm-Elementes (28) rotationssymmetrisch sind und in der Kontaktzone, vorzugsweise in Form einer Verzahnung, derart ausgebildet sind, dass sie in der Verspannrichtung annähernd reibungsfrei gegeneinander verschiebbar aber nicht verdrehbar sind.

6. Klemmverbindung (10) nach einem der Ansprüche 1 - 5, dadurch gekennzeichnet, dass das Verbindungselement (15) zylindrisch, rechteckig oder andersförmig ausgebildet ist und an seinen Stirnflächen je eine gleichachsige Gewindeschraube (16) hat, auf letzterer jeweils ein kappenförmiges Klemmelement (18) angeordnet ist, das eine Klemmkraft (Fk) auf den dazwischengeklemmten Längsträger (12,14) ausübt und gegenüberliegend mit seiner Auflagefläche (17) parallel zur Verspannrichtung an der Auflagefläche des Verbindungselementes (15) aufliegt.

7. Klemmverbindung (10) nach Anspruch 6, dadurch gekennzeichnet, dass die Auflageflächen (17;35''') des Klemmelementes (18;39) und des Verbindungselementes (15,35) zylindrisch, eben oder andersförmig ausgebildet sind.

8. Klemmverbindung (10,20,30,40,50) nach einem der Ansprüche 1 - 7, dadurch gekennzeichnet, dass das Verbindungselement (15,25,35,45,55) im Bereich seines Längskörpers eine seitliche, den Längsträger (12) lagernde Ausnehmung (35'') aufweist, die eine annähernd halbrunde Ausbildung hat, die zusammen mit dem Klemmelement (38) eine Dreipunktauflage in verspanntem Zustand des Längsträgers (22) bewirkt.

9. Klemmverbindung (30) nach Anspruch 8, dadurch gekennzeichnet, dass der Längsträger (12) rohrförmig ausgebildet ist, damit er zwecks Erhöhung der Klemmwirkung eine gewisse Deformation und Anpassung an die Ausnehmung (35'') des Verbindungselementes (35) erfährt.

10. Klemmverbindung (20) nach einem der Ansprüche 1 - 5, dadurch gekennzeichnet, dass für ein eine Pedikelschraube (23) aufweisendes zylindrisches Verbindungselement (25) mit zwei Ausnehmungen (25') für je einen Längsträger (22,24) zwei auf diesem axial verschiebbar geführten ringförmige Klemmelemente (28,29) vorgesehen sind, wobei die Verspannmittel (26,26') mit dem einen Klemmelement (28), diese mit dem oberen Konstruktionselement (22), das seinerseits mit dem unteren Klemmelement (29) zur Anlage kommt und letzteres auf den im Verbindungselement (25) aufliegenden unteren Längsträger (24) drückt.

11. Klemmverbindung (10) nach einem der Ansprüche 1 - 10, dadurch gekennzeichnet, dass die Verspannmittel (16,16') eine mit dem Verbindungselement (15) koaxial befestigte Gewindeschraube (16) sowie eine am jeweiligen Klemmelement (18) angreifende Mutter (16') umfassen.

12. Klemmverbindung (10,20) nach einem der Ansprüche 1 - 11, dadurch gekennzeichnet, dass die Gewindeschraube (16), respektive das Verbindungselement (15,25) eine dem Längsträger (12) zugeordnete quere Nut (15'') aufweist.

13. Klemmverbindung (10) nach einem der Ansprüche 1 - 12, dadurch gekennzeichnet, dass die Lage des Längsträgers (12) zum Verbindungselement (15) durch die Ausrichtung der Ausnehmung (15'') für den Längsträger (12) gegeben ist.

14. Klemmverbindung (10) nach einem der Ansprüche 1 - 13, dadurch gekennzeichnet, dass die Ausnehmung (25') derart ausgebildet ist, dass der Träger (22) bis zu einem bestimmten Winkel gegenüber dem Verbindungselement (25) verdrehbar ist.

15. Klemmverbindung (10,20,30,40) nach einem der Ansprüche 1 - 14, dadurch gekennzeichnet, dass die Lage des Längsträgers (22,14) zum Verbindungselement (25,35,45) durch eine verdrehsichere Gestaltung der Innenfläche des Klemmelementes (28,39,49) und der Aussenfläche des Verbindungselementes (25,35,45), vorzugsweise durch Längsverzahnung oder durch eine nicht kreiszylindrische Geometrie des Verbindungselementes und des Klemmelementes, vorzugsweise in polygonaler Form, gegeben ist.

16. Klemmverbindung (60) nach einem der Ansprüche 1 - 15, dadurch gekennzeichnet, dass das Verbindungselement (65) wenigstens an einer seiner Stirnseiten eine den Längsträger (12) lagernde Ausnehmung (65') und eine darin koaxiale Gewindeschraube (66) als Verspannmittel hat, auf letzterer ein Klemmelement (68) angeordnet ist, welches in die Ausnehmung (65') hineinragt, einerseits eine parallel zur Gewindeschraube (66) wirkende Klemmkraft auf einen dazwischengeklemmten Längsträger (12) ausübt und andererseits mit seiner äusseren Auflagefläche an einer inneren des Verbindungselementes (65) parallel zur Verspannrichtung aufliegt und damit eine Auflagekraft senkrecht zur Klemmkraft bewirkt.

17. Klemmverbindung (60) nach Anspruch 16, dadurch gekennzeichnet, dass die Ausnehmung (65') des Verbindungselementes (65) annähernd zylindrisch mit einem aussermittigen Schlitz für den Längsträger (12) ausgebildet ist, während das Klemmelement (68) auf der der Spannfläche abgekehrten Seite eine korrespondierende Ausbildung (65'') zur Ausnehmung (65') hat.

18. Klemmverbindung (60) nach Anspruch 17, dadurch gekennzeichnet, dass die Ausnehmung (65') und die Ausbildung (65'') in einem Radius oder einer schiefen Ebene enden.

## Claims

1. Clamp connection (10,20) for fixation of a longitudinal bar (12,14,22,24), in particular of an osteosynthetic fixation device, with
A) a clamping component (18, 28, 29, 38, 48, 49, 58, 68) having an axial bore through-hole and a recess for receiving the longitudinal bar (12,14,22,24);
B) a connection component (15, 25, 35, 45, 55, 65) with a basic body and axially disposed longitudinal body introducible into the bore through-hole of the clamping component (18, 28, 29, 38, 48, 49, 58, 68), whereby
C) the connection component (15, 25, 35, 45, 55, 65) has a contacting surface (15',25'',35'',65''') which can be brought in lateral contact with a contacting surface (18',28',39',68') of the clamping component (18, 28, 29, 38, 48, 49, 58, 68);
D) bracing means (16,16',26,26',36,36',46,46',56,56',66,66') are provided which are arranged displaceably on the longitudinal body of the connection component (15, 25, 35, 45, 55, 65) and braceable against the latter;
E) the clamping component (18, 28, 29, 38, 48, 49, 58, 68) is pushing the longitudinal bar (12,14,22,24) introduced in its recess against the basic body of the connection component (15, 25, 35, 45, 55, 65) and simultaneously pressing against each other the contacting surfaces (15',25'',35''',65'''; 18',39',68',28') which are standing in lateral contact;
F) the clamping component (18, 28, 29, 38, 48, 49, 58, 68) is pressed against the connection component (15, 25, 35, 45, 55) by the moment created by the bracing power (Fv) with a bearing power (Fa) acting perpendicular to the bracing power (Fv), which creates a friction force (Fr) between the clamping and the connection component, which alone is reducing the bracing power (Fv) on the longitudinal bar (12, 14, 22, 24); and
G) the connection component (15, 25, 35, 45, 55) has a groove-like recess (19;25') for receiving the longitudinal bar (12, 14, 22, 24).

2. Clamp connection (10,20) according to claim 1, characterized by the fact that in order to achieve the maximum possible clamping power (Fk) on the longitudinal bar (12, 14, 22, 24) the ratio of the distance (a) between the axis of the longitudinal bar (12, 14, 22, 24) and the axis of the bracing means (16, 16', 26, 26') to the distance (b) between the axis of the longitudinal bar (12, 14, 22, 24) and the line of action of the bearing power (Fa) is less than 1.

3. Clamp connection (10,20) according to claim 2, characterized by the fact that the distance (b) is larger than the half-diameter (height) of the longitudinal bar (12, 14, 22, 24).

4. Clamp connection (10) according to one of the claims 1 - 3, characterized by the fact that the contacting surfaces (18'; 15') of the connection component (15) and clamping component (18) are smooth so that they can slide together more or less without friction.

5. Clamp connection (20,30) according to one of the claims 1 - 3, characterized by the fact that the contacting surfaces (28', 25', 32) of the connection component (25, 35) and the clamping component (28) are symmetrically rotatable, and in the contact zone they are designed, preferably in the form of toothing, in such a manner that they can slide against each other in the direction of the bracing with practically no friction, but cannot be turned.

6. Clamp connection (10) according to one of the claims 1 - 5, characterized by the fact that the connection component (15) is cylindrical or rectangular or has some other shape, and each of its front ends has a threaded screw (16) having the same axis, with each threaded screw (16) having a cap-shaped gripping component (18), and that a clamping power (Fk) is exerted on the longitudinal bar (12,14) clamped between them and rests opposite with its bearing surface (17) parallel to the direction of bracing on the bearing surface of the connection component (15).

7. Clamp connection (10) according to claim 6, characterized by the fact that the bearing surfaces (17;35''') of the clamping component (18;39) and the connection component (15;35) are cylindrical or flat or have some other shape.

8. Clamp connection (10,20,30,40,50) according to one of the claims 1 - 7, characterized by the fact that the connection component (15,25,35,45,55) has a lateral recess (35'') in the region of its longitudinal body that is approximately semi-circular, and which together with the clamping component (38) creates a three-point bearing when the longitudinal bar (22) is braced.

9. Clamp connection (30) according to claim 8, characterized by the fact that the longitudinal bar (12) is tubular and thus, in order to increase the clamping effect, it undergoes a certain deformation and adaptation to the recess (35'') of the clamp component (35).

10. Clamp connection (20) according to one of the claims 1 - 5, characterized by the fact that for a cylindrical connection component (25) having a pedicle screw (23) and two recesses (25'), each of which holds a longitudinal bar (22, 24), there are two annular clamping components (28, 29) that can be slid axially on the longitudinal bar (22, 24), whereby the bracing element (26, 26') comes into contact with one clamping component (28) and this clamping component (28) comes into contact with the top longitudinal bar (22), which in turn comes into contact with the bottom clamping component (29) and presses the bottom clamping component (29) on the bottom longitudinal bar (24) resting on the connection component (25).

11. Clamp connection (10) according to one of the claims 1 - 10, characterized by the fact that the bracing means (16,16') include a threaded screw (16) coaxially attached to the connection component (15) and a nut (16') that engages on the appropriate clamping component (18).

12. Clamp connection (10,20) according to one of the claims 1 - 11, characterized by the fact that the threaded screw (16) or the connection component (15, 25) has a groove (15'') positioned crossways to the longitudinal bar (12).

13. Clamp connection (10) according to one of the claims 1 - 12, characterized by the fact that the position of the longitudinal bar (12) to the connection component (15) is determined by the alignment of the recess (15'') for the longitudinal bar (12).

14. Clamp connection (10) according to one of the claims 1 - 13, characterized by the fact that the recess (25') is designed in such a way that the longitudinal bar (22) is rotatable with regard to the connection component (25) to a certain angle.

15. Clamp connection (10,20,30,40) according to one of the claims 1 - 14, characterized by the fact that the position of the longitudinal bar (22, 14) to the connection component (25, 35, 45) is determined by a non-turning design of the inner surface of the clamping component (28, 39, 49) and the outer surface of the connection component (25, 35, 45) is determined preferably by longitudinal toothing or by a non-circular-cylindrical geometry of the connection component and the clamping component preferably in polygonal shape.

16. Clamp connection (60) according to one of claims 1 - 15, characterized by the fact that the connection component (65) has on at least one of its front ends, as bracing, a recess (65') housing the longitudinal bar (12) and a coaxial threaded screw (66) therein, with a clamping component (68) being positioned on the latter, which said clamping component (68) projects into the recess (65'), exerts on the longitudinal bar (12) clamped in between them a clamping power that acts parallel to the threaded screw (66) on the one hand, and on the other hand rests via its external bearing surface on an internal bearing surface of the connection component (65) parallel to the direction of bracing, and thereby creates a bearing force perpendicular to the clamping force.

17. Clamp connection (60) according to claim 16, characterized by the fact that the recess (65') of the connection component (65) is more or less cylindrical and has an off-center slot for the longitudinal bar (12), while the clamping component (68) has, on the side turned away from the bearing surface, a formation (65'') that corresponds to the recess (65').

18. Clamp connection (60) according to claim 17, characterized by the fact that the recess (65') and the formation (65'') end in a radius or an oblique plane.

## Revendications

1. Raccord à pinçage (10, 20) pour la fixation d'un support longitudinal (12, 14, 22, 24), en particulier d'un dispositif de fixation pour ostéosynthèse, comportant:
A) un élément de pinçage (18, 28, 29, 38, 48, 49, 58, 68) traversé par un alésage axial, et qui possède une découpe de réception du support longitudinal (12, 14, 22, 24);
B) un élément de liaison (15, 25, 35, 45, 55, 65) comportant un corps de base et un corps longitudinal disposé axialement et pouvant être guidé à travers l'alésage traversant l'élément de pinçage (18, 28, 29, 38, 48, 49, 58, 68), tandis que
C) l'élément de liaison (15, 25, 35, 45, 55, 65) possède une surface de contact (15', 25'', 35'', 65''') qui peut être amenée en contact latéral avec une surface de contact (18', 28', 39', 68') de l'élément de pinçage (18, 28, 29, 38, 48, 49, 58, 68);
D) des moyens de serrage (16, 16', 26, 26', 36, 36', 46, 46', 56, 56', 66, 66') sont prévus et sont disposés à coulissement sur le corps longitudinal de l'élément de liaison (15, 25, 35, 45, 55, 65) et peuvent être serrés contre ce dernier;
E) l'élément de pinçage (18, 28, 29, 38, 48, 49, 58, 68) repousse le support longitudinal (12, 14, 22, 24) inséré dans sa découpe contre le corps de base de l'élément de liaison (15, 25, 35, 45, 55, 65) et en même temps comprime l'une contre l'autre les surfaces de contact (15', 25''', 35''', 65'''; 18', 39', 68', 28') placées en contact latéral;
F) l'élément de pinçage (18, 28, 29, 38, 48, 49, 58, 68) est alors repoussé par le couple créé par la force de serrage (Fv) contre l'élément de liaison (15, 25, 35, 45, 55) avec une force d'appui (Fa) agissant perpendiculairement à la force de serrage (Fv), et qui a pour effet une force de frottement (Fr) entre l'élément de pinçage et l'élément de liaison, cette force étant la seule qui réduise la force de serrage (Fv) exercée sur l'élément de construction (12, 14, 22, 24); et
G) l'élément de liaison (15, 25, 35, 45, 55) présente une découpe (19; 25') en forme de rainure pour la réception du support longitudinal (12, 14, 22, 24).

2. Raccord à pinçage (10, 20) selon la revendication 1, caractérisé en ce que pour atteindre une force de pinçage (Fk) aussi grande que possible s'exerçant sur le support longitudinal (12, 14, 22, 24), le rapport de l'écart (a) entre l'axe du support longitudinal (12, 14, 22, 24) et celui du moyen de serrage (16, 16', 26, 26') à l'écart (b) entre l'axe du support longitudinal (12, 14, 22, 24) et la ligne d'action de la force d'appui (Fa) valent moins que 1.

3. Raccord à pinçage (10, 20) selon la revendication 2, caractérisé en ce que l'écart (b) est supérieur au demi-diamètre du support longitudinal (12, 14, 22, 24).

4. Raccord à pinçage (10) selon l'une quelconque des revendications 1-3, caractérisé en ce que les surfaces de contact (18'; 15') de l'élément de liaison (15) et de l'élément de pinçage (18) sont de conformation lisse dans la zone de contact (17), en vue d'un coulissement mutuel approximativement sans frottement.

5. Raccord à pinçage (20, 30) selon l'une quelconque des revendications 1-3, caractérisé en ce que les surfaces de contact (28', 25', 32) de l'élément de liaison (25, 35) et de l'élément de pinçage (28) sont à symétrie de rotation, et en ce que dans la zone de contact, elles sont conformées de préférence sous la forme d'une cannelure, de telle sorte qu'elles puissent coulisser l'une par rapport à l'autre approximativement sans frottement dans la direction du serrage, mais qu'elles ne puissent pas tourner.

6. Raccord à pinçage (10) selon l'une quelconque des revendications 1-5, caractérisé en ce que l'élément de liaison (15) est de conformation cylindrique, rectangulaire ou autre, et en ce que sur ses surfaces frontales il possède chaque fois une vis filetée (16) de même axe, sur chacune desquelles est disposé un élément de pinçage (18) en forme de bonnet, qui exerce une force de pinçage (Fk) sur le support longitudinal (12, 14) pincé entre eux, et qui de chaque côté repose par sa surface d'appui (17), parallèlement à la direction de serrage, sur la surface d'appui de l'élément de liaison (15).

7. Raccord à pinçage (10) selon la revendication 6, caractérisé en ce que les surfaces d'appui (17; 35''') de l'élément de pinçage (18; 39) et de l'élément de liaison (15, 35) sont cylindriques, planes ou d'une autre forme.

8. Raccord à pinçage (10, 20, 30, 40, 50) selon l'une quelconque des revendications 1-7, caractérisé en ce que l'élément de liaison (15, 25, 35, 45, 55) présente dans la région de son corps longitudinal une découpe latérale (35'') logeant le support longitudinal (12) et qui présente la forme approximative d'un demi-cercle qui forme avec l'élément de pinçage (38) un appui à trois points lorsque le support longitudinal (22) est serré.

9. Raccord à pinçage (30) selon la revendication 8, caractérisé en ce que le corps longitudinal (12) est conformé en forme de tube pour être soumis à une certaine déformation et adaptation à la découpe (35'') de l'élément de liaison (35) en vue d'une augmentation de l'action de pinçage.

10. Raccord à pinçage (20) selon l'une quelconque des revendications 1-5, caractérisé en ce que pour un élément de liaison (25) cylindrique présentant une vis à pédicule (23) et comportant deux découpes (25'), chacune pour un support longitudinal (22, 24), deux éléments de pinçage (28, 29) de forme annulaire guidés à coulissement axial sur ce dernier sont prévus, les moyens de serrage (26, 26') repoussant un des éléments de pinçage (28), et celui-ci avec l'élément supérieur de construction (22), qui de son côté vient se poser sur l'élément inférieur de pinçage (29), et ce dernier repoussant le support longitudinal inférieur (24) disposé dans l'élément de liaison (25).

11. Raccord à pinçage (10) selon l'une quelconque des revendications 1-10, caractérisé en ce que les moyens de serrage (16, 16') comportent une vis filetée (16) fixée coaxialement sur l'élément de liaison (15), ainsi qu'un écrou (16') s'accrochant sur chaque élément de pinçage (18).

12. Raccord à pinçage (10, 20) selon l'une quelconque des revendications 1-11, caractérisé en ce que la vis filetée (16), respectivement l'élément de liaison (15, 25), présente une rainure (15'') transversale associée au support longitudinal (12).

13. Raccord à pinçage (10) selon l'une quelconque des revendications 1-12, caractérisé en ce que la position du support longitudinal (12) par rapport à l'élément de liaison (15) est fournie par l'orientation de la découpe (15'') prévue pour le support longitudinal (12).

14. Raccord à pinçage (10) selon l'une quelconque des revendications 1-13, caractérisé en ce que la découpe (25') est conformée de telle sorte que le support (22) puisse être tourné d'un angle défini par rapport à l'élément de liaison (25).

15. Raccord à pinçage (10, 20, 30, 40) selon l'une quelconque des revendications 1-14, caractérisé en ce que la position du support longitudinal (22, 14) par rapport à l'élément de liaison (25, 35, 45) est fournie par une conformation de la surface interne de l'élément de pinçage (28, 39, 49) et de la surface externe de l'élément de liaison (25, 35, 45) qui empêche ces surfaces de tourner, de préférence par une cannelure longitudinale ou par une géométrie non cylindrique circulaire de l'élément de liaison et de l'élément de pinçage, de préférence sous la forme d'un polygone.

16. Raccord à pinçage (60) selon l'une quelconque des revendications 1-15, caractérisé en ce que l'élément de liaison (65) présente au moins sur ses côtés frontaux une découpe (65') logeant le support longitudinal (12) et une vis filetée (66) coaxiale dans cette découpe (65') et servant de moyen de serrage, un élément de pinçage (68) étant disposé sur cette dernière et pénétrant dans la découpe (65'), et exerçant d'une part une force de pinçage agissant parallèlement à la vis filetée (66) sur le support longitudinal (12) pincé en position intermédiaire, et reposant d'autre part par sa surface externe d'appui sur une surface interne de l'élément de liaison (65), parallèlement à la direction de serrage, et exerçant ainsi une force d'appui perpendiculaire à la force de pinçage.

17. Raccord de pinçage (60) selon la revendication 16, caractérisé en ce que la découpe (65') de l'élément de liaison (65) est de conformation approximativement cylindrique avec une fente externe pour le support longitudinal (12), tandis que l'élément de pinçage (68) présente sur le côté éloigné de la surface de serrage une conformation (65'') correspondant à la découpe (65').

18. Raccord à pinçage (60) selon la revendication 17, caractérisé en ce que la découpe (65') et la conformation (65'') se terminent dans un rayon ou un plan incliné.
